(19) Europäisches Patentamt
European Patent Office
Office européen des brevets

(11) Veröffentlichungsnummer: 0 013 386
B1

(12) EUROPÄISCHE PATENTSCHRIFT

(45) Veröffentlichungstag der Patentschrift: 09.03.83

(51) Int. Cl.³: C 07 D 285/12

(21) Anmeldenummer: 79105248.3

(22) Anmeldetag: 18.12.79

# (54) Verfahren zur Herstellung von 1,3,4-Thiadiazolen.

(30) Priorität: 10.01.79 DE 2900706

(43) Veröffentlichungstag der Anmeldung: 23.07.80 Patentblatt 80/15

(45) Bekanntmachung des Hinweises auf die Patenterteilung: 09.03.83 Patentblatt 83/10

(84) Benannte Vertragsstaaten: BE CH DE FR GB

(56) Entgegenhaltungen:
DE - A - 2 447 387
DE - A - 2 526 308

(73) Patentinhaber: BASF Aktiengesellschaft
Carl-Bosch-Strasse 38
D-6700 Ludwigshafen (DE)

(72) Erfinder: Hagen, Helmut, Dr. Dipl.-Ing.
Max-Slevogt-Strasse 17E
D-6710 Frankenthal (DE)
Erfinder: Kohler, Rolf-Dieter, Dr. Dipl.-Chem.
Speyerer Strasse 3
D-6800 Mannheim 23 (DE)

EP 0 013 386 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 1,3,4-Thiadiazolen

Die Erfindung betrifft ein Verfahren zur Herstellung von 1,3,4-Thiadiazolen durch Umsetzung von 2-Alkyl-1,3,4-thiadiazolen mit Oxalsäurediestern und dann gewünschtenfalls Umsetzung der gebildeten 1,3,4-Thiadiazolyl-(2)-brenztraubensäureester mit Halogen oder Hypohalogeniten bei Temperaturen bis 60°C.

Die Herstellung der Mono- und Dichloralkyl-1,3,4-thiadiazole und 1,3,4-Thiadiazolyl-(2)-brenz traubensäureester wurde bisher noch nicht beschrieben. Arbeiten über die Herstellung chlormethylierter, heterocyclischer Verbindungen, die gewisse Rückschlüsse auf das Verhalten dieser 1,3,4-Thiadiazole zu ziehen erlauben, ergeben ein uneinheitliches Bild: 2-Alkylthiophen wird durch Chlor nach den üblichen Methoden der Chlorierung von Alkylbenzolen in der Seitenkette fast völlig im Kern chloriert. 3-Methylthiophen kann nur mit Phosphortrichlorid im Sonnenlicht zu einem geringeren Teil an der Methylgruppe chloriert werden. Spezielle Methoden wie die Verwendung von N-Bromsuccinimid und Benzoylperoxid führen nicht zur Herstellung von Tribrommethylverbindungen, sondern von Monobrommethylverbindungen. Zu Monochlormethyl-thiophen und 2,5-Bis(chlormethyl)-thiophen gelangt man durch Chlormethylierung von Thiophen mit Formaldehyd und Chlorwasserstoff [Hartough, Thiophene and its Derivatives (Interscience Publ., N.Y., 1952), Seiten 186—188]. 2,5-Dimethylthiophen ergibt bei der Chlorierung 3,4-Dichlor-2,5-dimethylthiophen; die Verbindung kann zwar in der Seitenkette bromiert werden, die Bromierung führt aber nur zu 3,4-Dichlor-2,5-bis(dibrommethyl)-thiophen (Hartough, loc. cit., Seite 183).

Für die Herstellung von Di(trichlormethyl)-1,3,4-oxadiazol wird die Umsetzung von Ti(trichloracetyl)-hydrazin mit Phosphorpentachlorid empfohlen; eine direkte Chlorierung ist nicht beschrieben [Wiley, 5- and 6-Membered Compounds with Nitrogen and Oxygen (Interscience Publ., N.Y., 1962), Seite 265]. Die direkte Chlorierung oder Bromierung der Methylgruppen von Isoxazolen führt zu ganz oder teilweise kernhalogenierten Produkten bzw. zu uneinheitlichen Gemischen von Halogenierungsprodukten (Wiley, loc. cit., Seite 49).

Es ist aus der deutschen Offenlegungsschrift 22 53 863 bekannt, daß man 2,5-Bistrichlormethyl-1,3,4-thiadiazol erhält, wenn man 2,5-Dimethyl-1,3,4-thiadiazol mit Chlor in Gegenwart einer organischen Säure umsetzt.

Die Umsetzung von 1,2-Dimethyl-5-nitroimidazol mit Oxalsäureäthylesterchlorid liefert in Gegenwart von Triäthylamin während 22 Stunden unter Verwendung größerer Mengen an Äther 1-Carbäthoxy-2-(1-methyl-5-nitro-2-imidazolyl)-vinyl-äthyloxalat in 54-prozentiger Ausbeute, das beim Behandeln mit Äthanol 1-Methyl-5-nitroimidazol-2-yl-brenztraubensäureäthylester in 43-prozentiger Ausbeute ergibt (J. Heterocycl., loc. cit., Seiten 904 und 905)

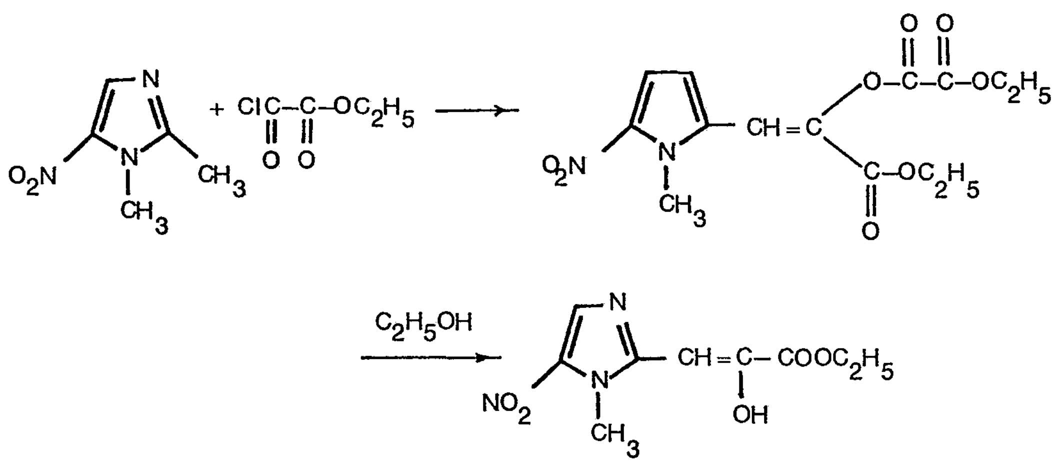


Es wurde nun gefunden, daß man 1,3,4-Thiadiazole der Formel I

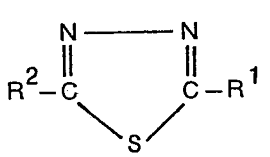
(I)

worin $R^1$ den Rest 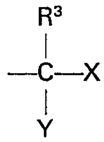 oder den Rest 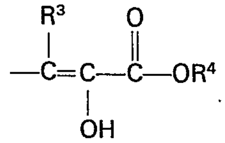

bedeutet, $R^2$ ein Wasserstoffatom, einen Alkylrest mit 1 bis 7 Kohlenstoffatomen, einen Alkenylrest mit 2 bis 7 Kohlenstoffatomen, einen Cycloalkylrest mit 5 bis 6 Kohlenstoffatomen, einen Aralkylrest mit 7 bis 12 Kohlenstoffatomen, einen Aralkenylrest mit 8 bis 12 Kohlenstoffatomen, einen Phenylrest bezeichnet, $R^3$ für einen Alkylrest mit 1 bis 7 Kohlenstoffatomen oder ein Wasserstoffatom steht, $R^4$ ein Wasserstoffatom, einen Alkylrest mit 1 bis 7 Kohlenstoffatomen, einen Alkenylrest mit 2 bis 7 Kohlenstoffatomen, einen Cycloalkylrest mit 5 bis 6 Kohlenstoffatomen, einen Aralkylrest mit 7 bis 12 Kohlenstoffatomen bedeutet, Y ein Wasserstoffatom oder ein Halogenatom bezeichnet, X für ein Halogenatom steht, vorteilhaft erhält, wenn man in einer ersten Stufe 2-Alkyl-1,3,4-thiadiazole der Formel II

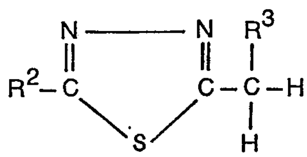

(II)

worin $R^2$ und $R^3$ die vorgenannte Bedeutung besitzen, mit Oxalsäurediestern der Formel III

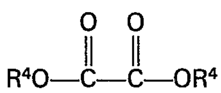

(III)

worin die einzelnen Reste $R^4$ gleich oder verschieden sein können und jeweils die vorgenannte Bedeutung besitzen, in Gegenwart von Alkoholaten umsetzt und gewünschtenfalls in einer zweiten Stufe die so erhaltenen 1,3,4-Thiadiazole der Formel Ia

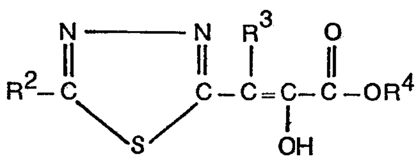

(Ia)

worin $R^2$, $R^3$ und $R^4$ die vorgenannte Bedeutung besitzen, oder ihre Metallenolate mit Halogen oder Hypohalogeniten zu den 1,3,4-Thiadiazolen der Formel Ib

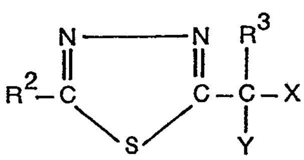

(Ib)

worin $R^2$, $R^3$, X und Y die vorgenannte Bedeutung besitzen, bei einer Temperatur von höchstens 60°C umsetzt.

Weiterhin wurde gefunden, daß man 1,3,4-Thiadiazole der Formel Ib

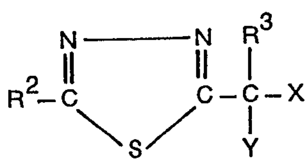

(Ib)

worin $R^2$, $R^3$, X und Y die vorgenannte Bedeutung besitzen, vorteilhaft erhält, wenn man 1,3,4-Thiadiazole der Formel Ia

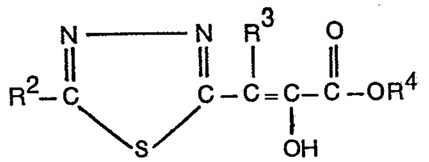

(Ia)

worin $R^2$, $R^3$ und $R^4$ die vorgenannte Bedeutung besitzen, oder ihre Metallenolate mit Halogen oder hypohalogeniten bei einer Temperatur von höchstens 60°C umsetzt.

Die Umsetzung kann für den Fall der Verwendung von 2,5-Dimethyl-1,3,4-thiadiazol, Oxalsäurediäthylester und Natriumhypochlorit durch die folgenden Formeln wiedergegeben werden:

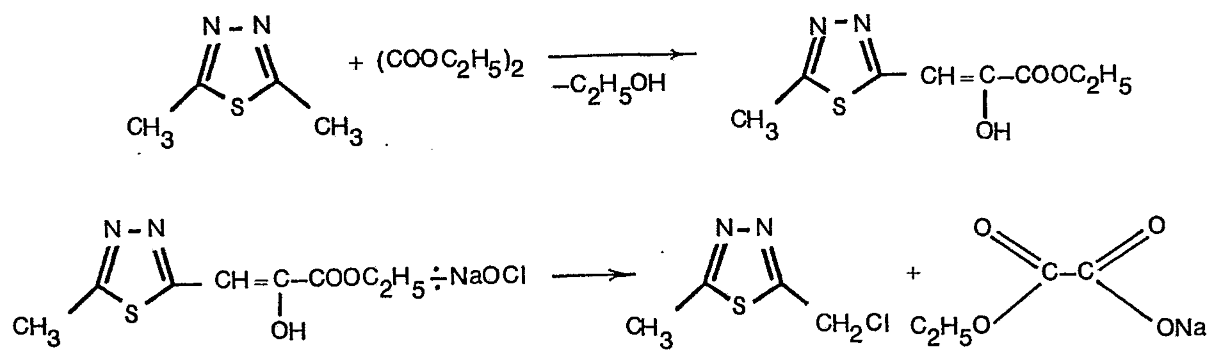


Das Verfahren nach der Erfindung liefert auf einfachem und wirtschaftlichem Wege die 1,3,4-Thiadiazole in guter Ausbeute und Reinheit. Oxalsäurediester sind technisch gut zugängliche Ausgangsverbindungen, der gleichzeitig entstehende Oxalsäuremonoester bzw. die durch Hydrolyse freiwerdende Oxalsäure kann isoliert und erneut zur Herstellung von Oxalsäurediestern eingesetzt werden. Alle diese vorteilhaften Ergebnisse sind im Hinblick auf den Stand der Technik überraschend, denn man hätte im Hinblick auf den Stand der Technik die Undurchführbarkeit der Halogenierung bzw. die Bildung heterogener Gemische zahlreicher Komponenten, bei der Umsetzung mit Oxalsäurediestern mindestens schlechte Ausbeuten und Reinheit der Stoffe Ia und insgesamt kein gerade im industriellen Maßstab durchführbares Verfahren erwarten müssen.

Die 2-Alkyl-1,3,4-thiadiazole II werden z.B. nach dem in Ber. dtsch. chem. Ges. *32*, 798 (1899) oder dem in der deutschen Offenlegungsschrift 21 32 019 beschriebenen Verfahren hergestellt. Der Ausgangsstoff II kann mit dem Ausgangsstoff III in stöchiometrischer Menge oder im Überschuß, vorzugsweise in einem Verhältnis von 1 bis 3 Mol Ausgangsstoff III je Mol Ausgangsstoff II, umgesetzt werden. Bevorzugte Stoffe Ib und Endstoffe I sind solche, in deren Formeln Y für ein Bromatom und insbesondere ein Chloratom steht oder ein Wasserstoffatom bezeichnet, X für ein Bromatom und insbesondere ein Chloratom steht.

So kommen beispielsweise folgende 2-Alkyl-1,3,4-thiadiazole als Ausgangsstoffe II in Betracht: 2-Methyl-, 2-Äthyl-, 2-Propyl-, 2-Pentyl-, 2-Butyl-, 2-Hexyl-, 2-Heptyl-1,3,4-thiadiazol; analoge in 5-Stellung durch die Äthyl-, Propyl-, Isopropyl-, Butyl-, Isobutyl-, sek.-Butyl-, tert.-Butyl-, Methyl-, Styryl-, Phenyläthyl-, Cyclohexyl-, Benzyl-, Phenyl-gruppe substituierte 1,3,4-Thiadiazole.

Beispielsweise sind folgende Oxalsäurediester als Ausgangsstoffe III geeignet: Der Dimethyl-Diäthyl-, Dipropyl-, Diisopropyl-, Dibutyl-, Diisobutyl-, Di-sek.-butyl-, Di-tert.-butyl-, Dicyclopentyl-, Dicyclohexyl-, Dibenzyl-, Di-phenyläthyl)-ester, Monomethyl-monoäthyl-ester der Oxalsäure.

Die Umsetzung in der ersten Stufe wird in Gegenwart von Alkoholaten, vorteilhaft in einer Menge von 0,5 bis 5, vorzugsweise von 1 bis 3 Äquivalenten Alkoholat, bezogen auf ein Mol Ausgangsstoff II, durchgeführt. Als Alkoholate werden zweckmäßig Erdalkalialkoholate und insbesondere Alkalialkoholate sowie entsprechende Gemische verwendet. Bevorzugt sind Natrium- und Kaliumalkoholate; unter den Alkoholaten sind Alkanolate vorteilhaft. Es kommen z.B. als Alkoholate in Frage: Natriummethylat, Natriumäthylat, Natriumpropylat, Natriumisopropylat, Natriumbutylat, Natriumisobutylat, Natrium-sek.-butylat, Natrium-tert.-butylat, Natriumäthylenglykolat, Natriumpropylen-(1,2)-glykolat, Natriumpropylen-(1,3)-glykolat, Natriumdiäthylenglykolat, Natriumtriäthylenglykolat, Natriumdipropylen-(1,2)-glykolat, Kaliummethylat, Kaliumäthylat, Kalium-n-propylat, Kaliumisopropylat, Kalium-n-butylat, Kalium-isobutylat, Kalium-sek.-butylat, Kalium-tert.-butylat, Kaliumäthylenglykolat, Kaliumpropylen-(1,2)-glykolat, Kaliumpropylen-(1,3)-glykolat, Kaliumdiäthylenglykolat, Kaliumtriäthylenglykolat, Kaliumdipropylen-(1,2)-glykolat; besonders bevorzugt sind vorgenannte Alkanolate mit 1 bis 4 Kohlenstoffatomen.

Die Umsetzung wird in der ersten Stufe im allgemeinen bei einer Temperatur von 0 bis 120, vorzugsweise von 20 bis 100°C, insbesondere bei 50 bis 90°C, drucklos oder zweckmäßig unter Druck, kontinuierlich oder diskontinuierlich durchgeführt. Man verwendet unter den Reaktionsbedingungen inerte Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, Diacetonalkohol, insbesondere solche mit 1 bis 4 Kohlenstoffatomen; und entsprechende Gemische. Zweckmäßig verwendet man das Lösungsmittel in einer Menge von 100 bis 10 000 Gewichtsprozent, vorzugsweise von 200 bis 1 500 Gewichtsprozent, bezogen auf Ausgangsstoff II.

Die erste Stufe der Reaktion kann wie folgt durchgeführt werden: Ein Gemisch von Ausgangsstoff II und III zusammen mit Alkoholat und Lösungsmittel wird während 0,5 bis 24 Stunden bei der Reaktionstemperatur gehalten. Man kann den Ausgangsstoff III oder den Ausgangsstoff II zusammen mit Lösungsmittel vorlegen und dann die anderen Komponenten zugeben. Aus dem Reaktionsgemisch wird der Stoff Ia in üblicher Weise, zweckmäßig durch Destillation, Behandeln des Rückstandes mit Säure wie wäßrige Salze- oder Schwefelsäure und Filtration, abgetrennt.

Man kann auch den Rückstand der Destillation, der die Metallenolate der Stoffe Ia enthält, abtrennen. Die Metallenolate der Stoffe Ia sind Stoffe der Formel IV

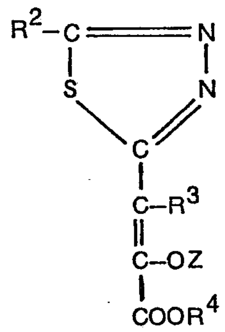

(IV)

worin $R^3$, $R^2$ und $R^4$ die vorgenannte Bedeutung besitzen und Z das Äquivalent eines Metallatoms, vorzugsweise eines Alkaliatoms, bezeichnet, Bevorzugt sind Kaliumenolate und insbesondere Natriumenolate IV. Diese Metallenolate IV können ebenfalls anstelle der Stoffe Ia in der zweiten Reaktionsstufe umgesetzt werden. Auch Gemische von Stoff Ia mit basischen, insbesondere alkalischen Verbindungen wie Natronlauge oder Natriumcarbonat, sind zweckmäßig, da so Stoff Ia als Enolat in eine zweckmäßig wäßrige Ausgangslösung gebracht wird. Der Stoff Ia kann auch in der tautomeren Ketoform

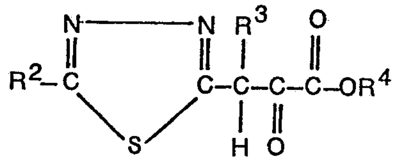

verwendet werden.

Stoff IV oder Stoff Ia kann in der zweiten Stufe des erfindungsgemäßen Verfahrens mit Halogen, vorzugsweise Brom, Jod und insbesondere Chlor, in stöchiometrischer Menge oder im Überschuß, vorzugsweise im Falle der Herstellung der Dihalogenverbindungen Ib ($X = Y =$ Halogen) in einem Verhältnis von 2 bis 2,5 Mol, im Falle der Herstellung der Monohalogenverbindungen Ib ($X =$ Halogen; $Y = H$) von 0,5 bis 1,5, vorzugsweise 1 bis 1,3 Mol Halogen, insbesondere $Cl_2$, je Mol Ausgangsstoff II, umgesetzt werden. In der zweiten Verfahrensstufe mit Halogen wird die Umsetzung im allgemeinen im sauren Medium durchgeführt, da ja Halogenwasserstoff gebildet wird. Zweckmäßig verwendet man daneben eine zusätzliche Säure, vorteilhaft in einer Menge von 1 bis 4, insbesondere von 2 bis 2,5 Äquivalenten Säure, im Falle der Verwendung von Metallenolaten IV in einer Menge von 2 bis 5, insbesondere von 3 bis 3,5 Äquivalenten Säure, bezogen auf 1 Mol Ausgangsstoff II bzw. ein Äquivalent Metallenolat IV. Es können anorganische oder organische Säuren verwendet werden. Anstelle einbasischer Säuren können auch äquivalente Mengen mehrbasischer Säuren zur Anwendung gelangen. Beispielsweise sind folgende Säuren geeignet: Chlorwasserstoff, Bromwasserstoff, Jodwasserstoff, Perchlorsäure, Schwefelsäure, Phosphorsäure, Borsäure, Salpetersäure, Sulfonsäuren wie Benzol- und p-Toluolsulfonsäure; aliphatische Carbonsäuren wie Chloressigsäure, Dichloressigsäure, Trichloressigsäure, Oxalsäure, Ameisensäure, Cyanessigsäure, Essigsäure, Propionsäure, Buttersäure, Isobuttersäure, Weinsäure, Citronensäure, $\beta$-Oxybuttersäure, Caprylsäure, Trimethylessigsäure, $\alpha$- bzw. $\beta$-Chlorpropionsäure, Bernsteinsäure, Isovaleriansäure, Valeriansäure, Glutarsäure, Adipinsäure; oder entsprechende Gemische. Die Säuren können in konzentrierter Form, im Gemisch miteinander und/oder mit einem Lösungsmittel, insbesondere Wasser, angewendet werden. Bei verdünnten, wäßrigen Säuren sind 15- bis 35-gewichtsprozentige Säuren, z.B. 20- bis 30-gewichtsprozentige Salzsäure, 10-

bis 50-, vorzugsweise 20- bis 30-gewichtsprozentige Schwefelsäure oder 50- bis 100-gewichtsprozentige Essigsäure, vorteilhaft. Bevorzugt sind Salzsäure, Schwefelsäure, Phosphorsäure, Ameisensäure, Essigsäure, Monochloressigsäure, Di-, Trichloressigsäure. Der pH der zweiten Stufe beträgt zwischen 0 und 7, vorzugsweise von 1 bis 6, insbesondere von 2 bis 3.

Als Ausgangsstoffe verwendet man anstelle von Halogenen in der zweiten Verfahrensstufe auch Hypohalogenite, in der Regel Hypochlorite und Hypobromite in wäßrigem Medium, in der Regel in Gestalt von entsprechenden wäßrigen, alkalischen Lösungen. Vorteilhaft gelangen wäßrige Lösungen von 1 bis 50 Gewichtsprozent Stoff Ia zur Anwendung. Die wäßrigen Hypohalogenitlösungen enthalten im allgemeinen von 5 bis 15, vorzugsweise von 12 bis 14 Gewichtsprozent Hypohalogenit, vorzugsweise Hypochlorit, und können zweckmäßig zusätzlich von 0,2 bis 2,5 Mol, vorzugsweise 1 bis 2,1 Mol Alkalihydroxid je Mol Hypohalogenit, vorzugsweise Hypochlorit, enthalten. Man setzt die Hypohalogenite im Falle der Herstellung der Monohalogenverbindungen Ib (X = Halogen; Y = H) in einer Menge von 1 bis 1,2, vorzugsweise von 1,05 bis 1,1, im Falle der Herstellung der Dihalogenverbindungen Ib (X = Y = Halogen) von 2 bis 2,5, vorzugsweise von 2,05 bis 2,2 Äquivalenten Hypohalogenit, bezogen auf ein Mol Stoff Ia, um. Der Äquivalenz wird das oben angegebene Formelschema zugrundegelegt, z.B. bedeuten ein Mol Natriumhypochlorit oder 1/2 Mol Calciumhypochlorit ein Äquivalent eines Mols Stoff Ia. Vorteilhaft verwendet man Calciumhypochlorit, Magnesiumhypochlorit, Bariumhypochlorit, Lithiumhypochlorit, vorzugsweise Kaliumhypochlorit und insbesondere Natriumhypochlorit. Im Ausgangsgemisch beider Ausgangsstoffe kommen im allgemeinen Mengen von zweckmäßig insgesamt 0,2 bis 2,5 Mol, vorzugsweise 1 bis 2,1 Mol Alkalihydroxid (nicht eingerechnet das im Hpohalogenit enthaltene Alkali), bezogen auf 1 Mol Stoff Ia, in Frage. Enthält die wäßrige Hypohalogenitlösung kein freies Alkalihydroxid, so werden am Anfang oder im Laufe der Umsetzung zweckmäßig von 0,2 bis 2, vorzugsweise 1 bis 2 Mol Alkalihydroxid pro Mol Hypohalogenit zugeführt. Bevorzugte Alkalihypohalogenite sind das Natrium- oder das Kaliumsalz. Die Umsetzung der zweiten Stufe wird im allgemeinen bei einer Temperatur von —5 bis +60°C, im Falle der Verwendung von Halogen vorzugsweise von 0 bis 50°C, insbesondere von 10 bis 25°C, im Falle der Verwendung von Hypohalogeniten vorzugsweise von —10 bis +40°C, insbesondere von —5 bis +30°C, mit Unterdurck, Überdruck oder drucklos, diskontinuierlich oder kontinuierlich, durchgeführt. Man verwendet unter den Reaktionsbedingungen inerte Lösungsmittel. Als Lösungsmittel kommen z.B. in Frage: die für die Umsetzung der ersten Verfahrensstufe genannten Lösungsmittel; bevorzugt Wasser; und entsprechende Gemische. Zweckmäßig verwendet man das Lösungsmittel in einer Menge von 100 bis 10 000 Gewichtsprozent, vorzurgsweise von 200 bis 1 500 Gewichtsprozent, bezogen auf Ausgangsstoff II.

Die Reaktion kann wie folgt durchgeführt werden: Ein Gemisch von Ausgangsstoff IV oder Ia mit a) Halogen, Lösungsmittel und zusätzliche Säure oder b) Hypohalogenit, Wasser und Alkalihydroxid, wird während 1 bis 24 Stunden bei der Reaktionstemperatur gehalten. Dann wird der Stoff Ib aus dem Gemisch in üblicher Weise, z.B. durch Filtration, abgetrennt.

Zweckmäßiger gibt man dem Ausgangsgemisch der zweiten Verfahrensstufe noch ein mit Wasser nicht oder nur wenig mischbares, den Endstoff Ib lösendes, unter den Reaktionsbedingungen inertes Lösungsmittel zu. Als Lösungsmittel kommen z.B. in Frage: Halogenkohlenwasserstoffe, insbesondere Chlorkohlenwasserstoffe, z.B. Tetrachloräthylen, 1,1,2,2- oder 1,1,1,2-Tetrachloräthan, Amylchlorid, Cyclohexylchlorid, Dichlorpropan, Methylenchlorid, Dichlorbutan, Isopropylbromid, n-Propylbromid, Butylbromid, Chloroform, Äthyljodid, Propyljodid, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol 1,10-Dibromdecan, 1,4-Dibrombutan; und entsprechende Gemische. Zweckmäßig verwendet man das Lösungsmittel in einer Menge von 400 bis 10 000 Gewichtsprozent, vorugsweise von 500 bis 3 000 Gewichtsprozent, bezogen auf Stoff Ia. Nach der Reaktion wird dann in üblicher Weise der Endstoff Ib, z.B. durch Filtration, Abtrennen der organischen Phase, Extraktion der wäßrigen Phase mit demselben Lösungsmittel und Destillation der Extrakte, abgetrennt.

Die nach dem Verfahren der Erfindung hergestellten Stoffe sind wertvolle Ausgangsstoffe für die Herstellung von Farbstoffen, Schädlingsbekämpfungsmitteln und Pharmazeutika. So können die Stoffe Ib z.B. durch Umsetzung mit Triphenylphosphin in die entsprechenden Phosphoniumsalze umgewandelt werden. Diese Salze lassen sich durch eine weitere Reaktion mit 1 Alkyl-5-nitroimidazolen-2-aldehyden in die in der deutschen Offenlegungsschrift 25 44 702 beschriebenen, gegen Trichomonaden wirksame Pharmazeutika umsetzen. Die erfindungsgemäßen Stoffe sind weiterhin Hilfsmittel für die Kunststoffindustrie, sowie wertvolle Ausgangsstoffe für die Herstellung von Hilfsmitteln, Fluoreszenzmitteln und Aufhellern.

Die in den folgenden Beispielen angeführten Teile bedeuten Gewichtsteile. Sie verhalten sich zu den Volumenteilen wie Kilogramm zu Liter.

## Beispiel 1

a) 5-Methyl-1,3,4-thiadiazol-2-yl-brenztraubensäureäthylester

Zu einer Lösung von 114 Teilen 2,5-Dimethyl-1,3,4-thiadiazol in 300 Volumenteilen Äthanol gibt man innerhalb von 30 Minuten bei 60°C eine Mischung aus 180 Teilen 30-gewichtsprozentiger

Natriummethylat-Lösung (in Methanol), 146 Teilen Oxalsäurediäthylester und 400 Volumenteilen Äthanol. Nach 2 1/2 stündigem Rühren bei 60°C wird Äthanol im Vakuum abdestilliert. Den Rückstand gibt man unter starkem Rühren in 700 Volumenteile 5-gewichtsprozentige Salzsäure. Nach Absaugen und Trocknen erhält man 150 Teile 5-Methyl-1,3,4-thiadiazol-2-yl-brenztraubensäureäthylester (70% der Theorie) vom Fp 172°C.

b) 2-Chlormethyl-5-methyl-1,3,4-thiadiazol

Man löst 214 Teile 5-Methyl-1,3,4-thiadiazol-(2)-yl-brenztraubensäureäthylester in 2 000 Volumenteilen Wasser unter Zugabe von 80 Teilen Natriumcarbonat und gibt die Lösung in eine Dosiervorrichtung. In einer zweiten Dosiervorrichtung legt man 720 Teile einer 13-gewichtsprozentigen Natriumhypochlorit-Lösung vor. Im Reaktor befindet sich ein auf 3°C abgekühltes Gemisch aus 15 Teilen Natriumhydroxid, 100 Volumenteilen Wasser und 1 500 Volumenteilen Methylenchlorid. Unter starkem Rühren werden die beiden Lösungen getrennt zugegeben, wobei die Temperatur nicht über 5°C ansteigt. Nach beendeter Zugabe rührt man das Gemisch 2 Stunden bei 0°C, filtriert vom ausgefallenen Salz und trennt die Methylenchloridphase ab. Die wäßrige Phase wird mit Methylenchlorid mehrmals ausgeschüttelt. Die vereinigten Extrakte trocknet man und entfernt das Lösungsmittel im Vakuum. Man erhält 110 Teile 2-Chlormethyl-5-methyl-1,3,4-thiadiazol (74% der Theorie) vom Fp 82°C (Ligroin).

Beispiel 2

a) 5-Styryl-1,3,4-thiadiazol-2-yl-brenztraubensäureäthylester

Zu einer auf 60°C erwärmten Suspension von 64 Teilen 2-Methyl-5-styryl-1,3,4-thiadiazol in 200 Teilen Äthanol gibt man eine Mischung aus 46 Teilen Oxalsäurediäthylester, 56 Teilen 30-gewichtsprozentigem Natriummethylat (in Methanol) und 100 Volumenteilen Äthanol. Nach 2 stündigem Rühren bei 60°C wird das Gemisch abgekühlt, filtriert, das Filtergut mit der gleichen Menge Wasser versetzt und mit 2n-Salzsäure auf pH 3 angesäuert. Der ausgefallene Feststoff wird abgesaugt und getrocknet. Man erhält 66 Teile 5-Styryl-1,3,4-thiadiazol-2-yl-brenztraubensäureäthylester (66% der Theorie) vom Fp 195°C.

b) 2-Chlormethyl-5-styryl-1,3,4-thiadiazol

Ein Gemisch von 15 Teilen 5-Styryl-1,3,4-thiadiazol-2-yl-brenztraubensäureäthylester, 1,5 Teilen Natriumhydroxid un 5,3 Teilen Natriumcarbonat, gelöst in 100 Volumenteilen Wasser, und 100 Volumenteilen Methylenchlorid wird auf 0°C abgekühlt. Bei dieser Temperatur gibt man unter starkem Rühren 18 Teile einer 13-gewichtsprozentigen Natriumhypochlorit-Lösung zu und rührt das Gemisch nach Zugabe weitere 2 Stunden bei 0°C. Das Gemisch wird dann vom ausgefallenen Salz abfiltriert und die Methylenchloridphase abgetrennt. Die wäßrige Phase wird wiederholt mit Methylenchlorid ausgeschüttelt, die vereinigten Extrakte werden getrocknet und im Vakuum eingeengt. Man erhält 7,5 Teile 2-Chlormethyl-5-styryl-1,3,4-thiadiazol (63% der Theorie) vom Fp 105°C (Äthanol).

Beispiel 3

2-Dichloromethyl-5-methyl-1,3,4-thiadiazol

Zu einer auf 10°C gekühlten Mischung aus 22 Teilen Natriumhydroxid in 250 Teilen Wasser, 760 Teilen einer 13-gewichtsprozentigen Natriumhypochloritlösung und 1 500 Volumenteilen Methylenchlorid gibt man bei dieser Temperatur eine Lösung aus 186 Teilen 5-Methyl-1,3,4-thiadiazol-(2)-yl-brenztraubensäure, 50 Teilen Natriumcarbonat in 1 500 Volumenteilen Wasser. Nach einstündigem Rühren bei 10°C wird das Gemisch vom ausgefallenen Salz abfiltriert, die Methylenchloridphase abgetrennt, getrocknet und eingeengt. Man erhält 120 Teile 2-Dichlormethyl-5-methyl-1,3,4-thiadiazol (65% der Theorie) vom Fp 50°C (Ligroin).

Beispiel 4

2-Dichlormethyl-5-methyl-1,3,4-thiadiazol

In eine Lösung von 21 Teilen 5-Methyl-1,3,4-thiadiazol-(2)-yl-brenztraubensäureäthylester in 100 Volumenteilen 50-gewichtsprozentiger Schwefelsäure werden bei 20°C 15 Teile Chlor eingeleitet. Nach beendeter Reaktion gießt man das Gemisch auf Eis und filtriert das ausgefallene 2-Dichlormethyl-5-methyl-1,3,4-thiadiazol ab. Das mehrmalige Ausschütteln der wäßrigen Phase mit Methylenchlorid ergibt zusätzliches 2-Dichlormethyl-5-methyl-1,3,4-thiadiazol. Man erhält insgesamt 112 Teile 2-Dichlormethyl-5-methyl-1,3,4-thiadiazol (65% der Theorie) vom Fp 49°C (Ligroin).

Beispiel 5

2-Brommethyl-5-methyl-1,3,4-thiadiazol

Man löst 214 Teile 5-Methyl-1,3,4-thiadiazol-(2)-yl-brenztraubensäureäthylester in 1 000 Teilen Wasser unter Zugabe von 80 Teilen Natriumcarbonat und gibt die Lösung in eine Dosiervorrichtung. In einer zweiten Dosiervorrichtung befinden sich 500 Teile einer auf 0°C gekühlten Natriumhypobromit-Lösung, die durch Zugabe von 160 Teilen Brom zu einer Lösung von 90 Teilen Natriumhydroxid in 250 Volumenteilen Wasser hergestellt wird. Im Reaktor befindet sich ein auf —3°C abgekühltes Gemisch

aus 20 Teilen Natriumhydroxid, 100 Volumenteilen Wasser und 1 500 Volumenteilen Methylenchlorid. Unter starkem Rühren werden die beiden Lösungen getrannt zugegeben, wobei die Temperatur nicht über 0°C ansteigt. Nach beendeter Zugabe rührt man das Gemisch eine Stunde bei 0°C, filtriert vom ausgefallenen Salz und trennt die Methylenchloridphase ab. Die wäßrige Phase wird mit Methylenchlorid mehrmals ausgeschüttelt. Die vereinigten Extrakte trocknet man und entfernt das Lösungsmittel im Vakuum. Man erhält 95 Teile 2-Brommethyl-5-methyl-1,3,4-thiadiazol (49% der Theorie) vom Fp 80°C (Ligroin).

Beispiel 6

2-Dibrommethyl-5-methyl-1,3,4-thiadiazol

Eine Mischung aus 500 Teilen einer aus 160 Teilen Brom, 90 Teilen Natriumhydroxid und 250 Volumenteilen Wasser hergestellten Natriumhypobromit-Lösung und 800 Volumenteilen Methylenchlorid wird auf —3°C abgekühlt. Bei dieser Temperatur gibt man unter starkem Rühren eine Lösung aus 93 Teilen 5-Methyl-1,3,4-thiadiazol-(2)-yl-brenztraubensäure, 25 Teilen Natriumhydroxid in 500 Volumenteilen Wasser zu und rührt die Mischung nach Zugabe weiter 0,5 Stunden bei —3°C. Das Gemisch wird dann vom ausgefallenen Salz abfiltriert und die Methylenchloridphase abgetrennt. Die wäßrige Phase wird wiederholt mit Methylenchlorid ausgeschüttelt, die vereinigten Extrakte werden getrocknet und im Vakuum vom Lösungsmittel befreit. Man erhält 45 Teile 2-Dibrommethyl-5-methyl-1,3,4-thiadiazol (33% der Theorie) vom Fp 55°C (Ligroin).

**Patentansprüche**

1. Verfahren zu Herstellung von 1,3,4-Thiadiazolen der Formel I

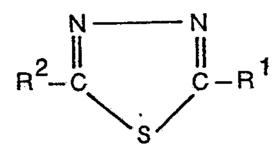 (I)

worin $R^1$ den Rest 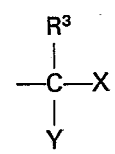 oder den Rest 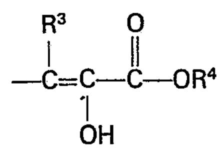

bedeutet, $R^2$ ein Wasserstoffatom, einen Alkylrest mit 1 bis 7 Kohlenstoffatomen, einen Alkenylrest mit 2 bis 7 Kohlenstoffatomen, einen Cycloalkylrest mit 5 bis 6 Kohlenstoffatomen, einen Aralkylrest mit 7 bis 12 Kohlenstoffatomen, einen Aralkenylrest mit 8 bis 12 Kohlenstoffatomen, einen Phenylrest bezeichnet, $R^3$ für einen Alkylrest mit 1 bis 7 Kohlenstoffatomen oder ein Wasserstoffatom steht, $R^4$ ein Wasserstoffatom, einen Alkylrest mit 1 bis 7 Kohlenstoffatomen, einen Alkenylrest mit 2 bis 7 Kohlenstoffatomen, einen Cycloalkylrest mit 5 bis 6 Kohlenstoffatomen, einen Aralkylrest mit 7 bis 12 Kohlenstoffatomen bedeutet, Y ein Wasserstoffatom oder ein Halogenatom bezeichnet, X für ein Halogenatom steht, dadurch gekennzeichnet, daß man in einer ersten Stufe 2-Alkyl-1,3,4-thiadiazole der Formel II

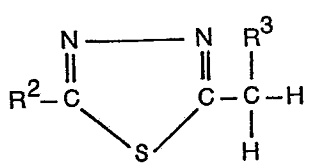 (II)

worin $R^2$ und $R^3$ die vorgenannte Bedeutung besitzen, mit Oxalsäurediestern der Formel III

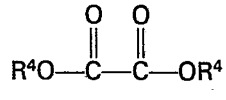 (III)

worin die einzelnen Reste $R^4$ gleich oder verschieden sein können und jeweils die vorgenannte Bedeutung besitzen, in Gegenwart von Alkoholaten umsetzt und gewünschtenfalls in einer zweiten Stufe die so erhaltenen 1,3,4-Thiadiazole der Formel Ia

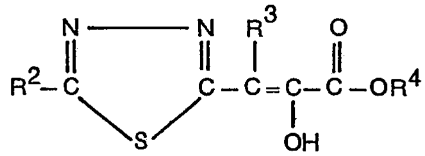 (Ia)

worin $R^2$, $R^3$ und $R^4$ die vorgenannte Bedeutung besitzen, oder ihre Metallenolate mit Halogen oder Hypohalogeniten zu den 1,3,4-Thiadiazolen der Formel Ib

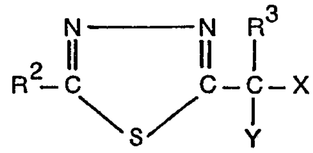 (Ib)

worin $R^2$, $R^3$, X und Y die vorgenannte Bedeutung besitzen, bei einer Temperatur von höchstens 60°C umsetzt.

2. Verfahren zur Herstellung von 1,3,4-Thiadiazolen der Formel Ib

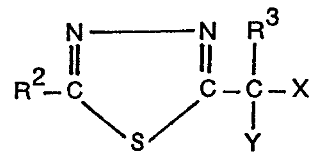 (Ib)

worin $R^2$, $R^3$, X und Y die vorgenannte Bedeutung besitzen, dadurch gekennzeichnet, daß man 1,3,4-Thiadiazole der Formel Ia

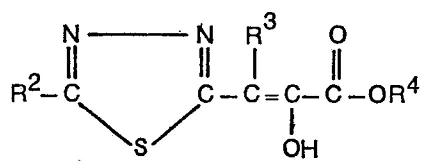 (Ia)

worin $R^2$, $R^3$ und $R^4$ die vorgenannte Bedeutung besitzen, oder ihre Metallenolate mit Halogen oder Hypohalogeniten bei einer Temperatur von höchstens 60°C umsetzt.

## Revendications

1. Procédé pour la préparation de 1,3,4-thiadiazoles de formule I

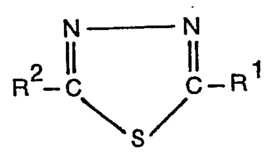 (I)

dans laquelle $R^1$ représente le radical 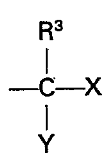 ou le radical 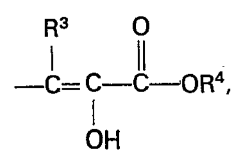

$R^2$ désigne un atome d'hydrogène, un radical alcoyle à 1—7 atomes de carbone, un radical alcényle à 2—7 atomes de carbone, un radical cycloalcoyle à 5—6 atomes de carbone, un radical aralcoyle à 7—12 atomes de carbone, un radical aralcényle à 8—12 atomes de carbone ou un radical phényle, $R^3$ est mis pour un radical alcoyle à 1—7 atomes de carbone ou pour un atome d'hydrogène, $R^4$ désigne un atome d'hydrogène, un radical alcoyle à 1—7 atomes de carbone, un radical alcényle à 2—7 atomes de carbone, un radical cycloalcoyle à 5—6 atomes de carbone ou un radical aralcoyle à 7—12 atomes de carbone, Y désigne un atome d'hydrogène ou un atome d'halogène et X est mis pour un atome d'halogène, caractérisé en ce que, dans un premier stade, on fait réagir, en présence d'alcoolates, des 2-alcoyl-1,3,4-thiadiazoles de formule II

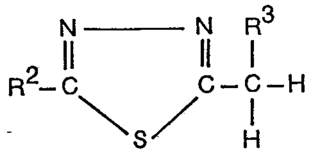

(II)

dans laquelle $R^2$ et $R^3$ ont les significations données ci-dessus, avec des diesters d'acide oxalique de formule III

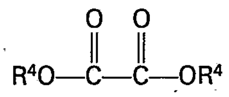

(III)

dans laquelle les différents radicaux $R^4$ peuvent être semblables ou différents et ont chacun la signification donnée ci-dessus, et en ce que, dans un second stade, si on le désire, on fait réagir, à une température de 60°C au maximum, les 1,3,4-thiadiazoles de formule Ia ainsi obtenus

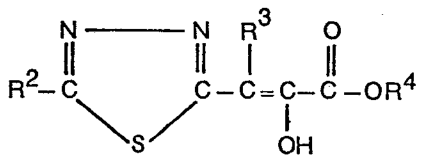

(Ia)

dans laquelle $R^2$, $R^3$ et $R^4$ ont les significations données ci-dessus, ou leurs énolates métalliques, avec des halogènes ou des hypohalogénites, pour obtenir les 1,3,4-thiadiazoles de formule Ib

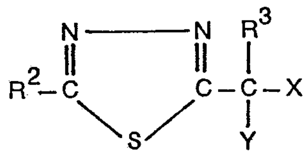

(Ib)

dans laquelle $R^2$, $R^3$, X et Y ont les significations données ci-dessus.

2. Procédé pour la préparation de 1,3,4-thiadiazoles de formule Ib

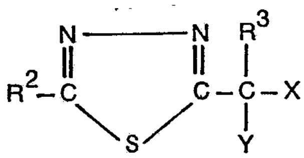

(Ib)

dans laquelle $R^2$, $R^3$, X et Y ont les significations données ci-dessus, caractérisé en ce qu'on fait réagir, à une température de 60°C au maximum, des 1,3,4-thiadiazoles de formule Ia

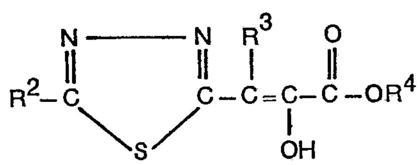

(Ia)

dans laquelle $R^2$, $R^3$ et $R^4$ ont les significations données ci-dessus, ou leurs énolates métalliques, avec des halogènes ou des hypohalogénites.

## Claims

1. Process for the preparation of 1,3,4-thiadiazoles of the formula I

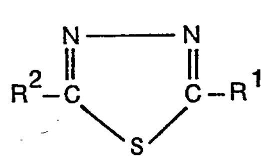

(I)

wherein $R^1$ denotes the radical 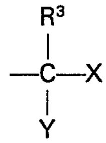 or the radical 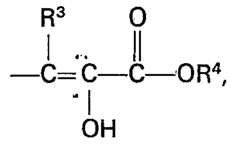,

$R^2$ designates a hydrogen atom, an alkyl radical of 1 to 7 carbon atoms, an alkenyl radical of 2 to 7 carbon atoms, a cycloalkyl radical of 5 to 6 carbon atoms, an aralkyl radical of 7 to 12 carbon atoms, an aralkenyl radical of 8 to 12 carbon atoms or a phenyl radical, $R^3$ represents an alkyl radical of 1 to 7 carbon atoms or a hydrogen atom, $R^4$ denotes a hydrogen atom, an alkyl radical of 1 to 7 carbon atoms, an alkenyl radical of 2 to 7 carbon atoms, a cycloalkyl radical of 5 to 6 carbon atoms or an aralkyl radical of 7 to 12 carbon atoms, Y designates a hydrogen atom or a halogen atom and X represents a halogen atom, wherein, in a first stage, 2-alkyl-1,3,4-thiadiazoles of the formula II

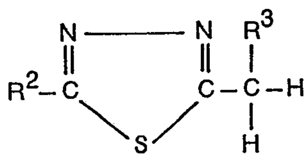 (II)

wherein $R^2$ and $R^3$ have the above meaning, are reacted with oxalic acid diesters of the formula III

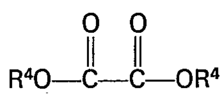 (III)

wherein the individual radicals $R^4$ can be identical or different and each has the above meaning, in the presence of alcoholates, and, if desired, in a second stage, the resulting 1,3,4-thiadiazoles of the formula Ia

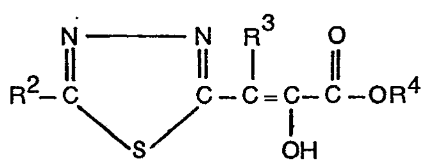 (Ia)

wherein $R^2$, $R^3$ and $R^4$ have the above meaning, or their metal enolates, are reacted with halogen or hypohalites at a temperature of at most 60°C, to give the 1,3,4-thiadiazoles of the formula Ib

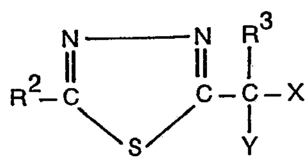 (Ib)

wherein $R^2$, $R^3$, X and Y have the above meaning.

2. Process for the preparation of 1,3,4-thiadiazoles of the formula Ib

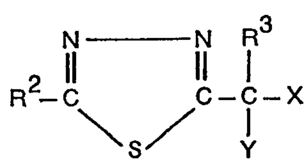 (Ib)

wherein $R^2$, $R^3$, X and Y have the above meaning, wherein 1,3,4-thiadiazoles of the formula Ia

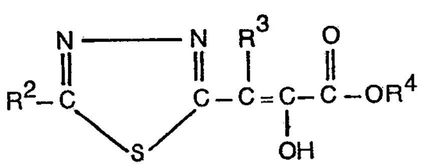 (Ia)

wherein $R^2$, $R^3$ and $R^4$ have the above meaning, or their metal enolates, are reacted with halogen or hypohalites at a temperature of at most 60°C.